# EUROPEAN PATENT SPECIFICATION

(11) **EP 1 887 870 B1**
(45) Date of publication and mention of the grant of the patent: **05.09.2018**
(21) Application number: 06772016.9
(22) Date of filing: 02.06.2006
(51) Int. Cl.: A01N 65/00, A01N 63/00, A61M 5/142

(54) **PREVASCULARIZED DEVICES AND RELATED METHODS**
VORVASKULARISIERTE VORRICHTUNGEN UND VERWANDTE VERFAHREN
DISPOSITIFS PRÉVASCULARISÉS ET MÉTHODES CONNEXES

(30) Priority: 02.06.2005 US 686706 P
(43) Date of publication of application: 20.02.2008
(73) Proprietor: Arizona Board of Regents on behalf of the University of Arizona, Tucson, AZ 85721-0300 (US)
(72) Inventor: WILLIAMS, Stuart, K., Tucson, AZ (US); HOYING, James, B., Tucson, AZ (US)
(74) Representative: Roberts, Peter David
(86) International application number: PCT/US2006/021542
(87) International publication number: WO 2006/130851

(56) References cited:
- EP-A1- 1 466 632
- WO-A1-93/08850
- WO-A2-00/35371
- US-A- 5 262 055
- US-A1- 2003 069 917
- US-B1- 6 884 427
- US-B2- 7 029 838
- US-B2- 7 052 829

## Description

### FIELD OF THE INVENTION

The present disclosure relates generally to devices for improving the efficacy of cell based therapies through use of prevascularized constructs, including devices and immunoisolation devices which significantly prolong the viability and function of implanted cells.

### BACKGROUND OF THE INVENTION

Diabetes is a group of diseases characterized by high levels of blood glucose resulting from defects in insulin production, insulin action or both. Presently, it is estimated that approximately 20.8 million people in the United States, or approximately 7% of the U.S. population, have diabetes, and incidence of the disease is growing.

The major types of diabetes include type 1 diabetes, type 2 diabetes, gestational diabetes, and pre-diabetes. Other types of diabetes can also result from specific genetic conditions, surgery, drugs, malnutrition, infections, and other illnesses. These "other" types of diabetes account for approximately 1-5% of all diagnosed cases of the disease.

Type 1 diabetes, also referred to as insulin-dependent diabetes mellitus (IDDM) or juvenile-onset diabetes, results when the body's immune system destroys insulin producing pancreatic beta cells. Type 1 diabetes accounts for approximately 5-10% of all diagnosed cases. Type 2 diabetes, also referred to as non-insulin dependent diabetes mellitus (NIDDM) or adult-onset diabetes, results from insulin resistance, combined with relative insulin deficiency. Type 2 diabetes represents approximately 90% of all diagnosed cases. It affects mostly people 45 or older and is associated with obesity and sedentary lifestyle.

Patients who have been diagnosed with type 1 or type 2 diabetes have significantly increased risk for developing other serious complications including cardiovascular disease, hypertension, stroke, limb amputation, retinoplasty, neuropathy, nephropathy, periodontal disease, complications during pregnancy and other complications including diabetic ketoacidosis and hypersmolar coma.

Gestational diabetes affects about 4% of all pregnant women and results from a form of glucose intolerance. Women who have had gestational diabetes have a 20-50% chance of developing another form of the disease within the following 5-10 years. Pre-diabetes is characterized by a blood glucose level that is higher than normal but still not high enough for a diagnosis of type 2 diabetes. People with pre-diabetes have impaired fasting glucose or impaired glucose tolerance or both. It is estimated that 41 million Americans suffer from a pre-diabetes condition, and are thus at increased risk for developing type 2 diabetes, heart disease and stroke.

Patients with type 1 diabetes must have insulin delivered via injection or pump in order to survive. In its early stages, some people with type 2 diabetes can manage the disease through a combination of drugs that increase pancreatic insulin, or act on the liver, muscle or intestine, plus lifestyle changes in diet and exercise. However, despite these efforts, 40% of all type 2 diabetic patients eventually require injections of insulin. Accordingly, the most promising treatment for both type 1 and type 2 diabetes may be the replacement of damaged pancreatic beta cells with intact functioning beta cells through beta islet transplantation.

Beta-cell replacement therapy via islet transplantation has reached the stage of clinical trials. However, there remain major limitations associated with the therapy - *i.e.* the loss of beta cell viability and function due to the lack of a blood supply to support cell viability. While whole organ pancreatic allografts have an expected graft survival of greater than 80% at one year, the risk of major postoperative morbidity and the risks of chronic immunosuppression limit this approach. Beta-cell transplantation offers several advantages over whole organ transplantation, including the ability to isolate and maintain islets from organs not suitable for whole organ transplantation, reduced morbidity and mortality, and finally, the opportunity to immuno-isolate the beta-cells and avoid the use of immuno-suppressive drugs. A significant barrier to the clinical utilization of beta cell transplants has been the lack of a host-derived blood supply to maintain the viability and thus the function of transplanted cells (De Vos P. et al. Efficacy of a prevascularized expanded polytetrafluoroethylene solid support system as a transplantation site for pancreatic islets. Transplantation 63: 824-830, 1997; Risbud M.V. and Bhonde R.R. Islet immunoisolation: experience with biopolymers. J Biomater Sci Polym Ed 12: 1243-1252, 2001; Vajkoczy P. et al. Angiogenesis and vascularization of murine pancreatic islet isografts. Transplantation 60: 123-127, 1995). In the field of biomaterial implants and peri-implant healing responses, the development of an avascular fibrous capsule provides the most germane example of the elements of angiostasis, angiogenesis and angioregresssion (Auerbach R. et al. Angiogenesis assays: a critical overview. Clin Chem 49: 32-40, 2003; Djonov V. et al. Vascular remodeling by intussusceptive angiogenesis. Cell Tissue Res 2003; Folkman J. Anti-angiogenesis: new concept for therapy of solid tumors. Am Surg 175: 409-416, 1972; Folkman J. Tumor angiogenesis. Adv Cancer Res 19: 331-358, 1974; Hoying J.B. et al. Heterogeneity in Angiogenesis. In: Genetics of Angiogenesis, edited by Hoying J.B. BIOS Scientific Publishers Ltd., 2003, p. 191-203; Ingber D.E. Mechanical signaling and the cellular response to extracellular matrix in angiogenesis and cardiovascular physiology. Circ Res 91: 877-887; 2002; Kale S. et al. Microarray analysis of in vitro pericyte differentiation reveals an angiogenic program of gene expression. FASEB J 19: 270-271; 2005; Pierce S. and Skalak T.C. Microvascular remodeling: a complex continuum spanning angiogenesis to arteriogenesis. Microcirculation 10: 99-111, 2003; Rifkin D.B. et al. The involvement of proteases and protease inhibitors in neovascularization. Acta Biol Med Ger 40: 1259-1263, 1981; Wahlberg E. Angiogenesis and arteriogenesis in limb ischemia. J Vasc Surg 38: 198-203, 2003). In view of the problems associated with the present cell replacement technologies, a need exists for devices and methods for use in cell replacement therapy that significantly improve the efficacy off cell therapies by prolong cell viability and function.

The inventors of the present invention have established new technologies to address these critical problems. Specifically, the inventors have developed a new cell based therapy for the generation of prevascularized tissue engineered constructs (Shepherd B.R. et al. Rapid perfusion and network remodeling in a microvascular construct after implantation. Arterioscler Thromb Vasc Biol 24: 898-904, 2004). In U.S. Patent 7,029,838 and U.S. Patent 7,052,829, the inventors previously described materials and methods for preparing prevascularized constructs which can be used to vascularize engineered tissue constructs or to revascularize damaged or diseased tissues or organs following implantation. The inventors have also developed a new generation of biomaterials that support extensive vascularization (Kidd K.R. et al. Angiogenesis and neovascularization associated with extracellular matrix-modified porous implants. Journal of Biomedical Materials Research 2: 366-377, 2002; Kidd K.R. et al. Angiogenesis and neovascularization associated with extracellular matrix-modified porous implants. J Biomed Mater Res 59: 366-377, 2002; Kidd K.R. and Williams S.K. Laminin-5-enriched extracellular matrix accelerates angiogenesis and neovascularization in association with ePTFE. J Biomed Mater Res A 69: 294-304, 2004). The combined cell and material construction is termed a Prevascularized Device or construct, or a Prevascularized ImmunoIsolation Device (PVID), and functions to significantly prolong cell viability and function *in vitro,* and specifically provides for long term function of transplanted beta islet cells in a subject. These constructs represent a preformed microcirculation that can be constructed from a patient's own fat-derived microvascular endothelial cells, avoiding the use of immuno-suppressive drugs (Williams S.K.Endothelial cell transplantation. Cell Transplant 4: 401-410, 1995).

EP1466632 describes an implantable pouch for seeding with insulin releasing cells to treat diabetes, the implantable pouch containing an opening for loading insulin releasing cells which opening may thereafter be closed and if desired, sealed shut. The device may contain reinforced porous foam and a lumen, the lumen containing an insert which may or may not be removed prior to implantation.

### SUMMARY OF THE INVENTION

The present invention is based on the discovery that a prevascularized construct or device, and/or prevascularized immunoisolation device, can be used to improve cell based therapies in a subject by significantly prolonging the viability and function of implanted cells. Specifically, the inventors have found that a hybrid system using a synthetic device or synthetic immunoisolation device in combination with a transplanted microcirculation allows implanted cells to function longer, thus, increasing their therapeutic efficacy. Additionally, use of this improved device eliminates the need for immuno-suppressive drugs and their attendant negative side effects. This discovery has broad implications in the treatment and prevention of diseases and disorders characterized by insufficient levels of one or more particular biologically active agents, including, but not limited to, diabetes.

Accordingly, the present invention provides an implantable device according to claim 1 for providing a biologically active agent to a subject, comprising a microvessel construct which is in contact with a biocompatible, semi-permeable pouch, wherein the pouch encapsulates cells or tissue capable of producing the biologically active agent. The invention encompasses microvessel constructs where the vessels of the microvessel construct are syngenic to the subject. The implanted cells or tissues may be allogenic or syngenic to the subject. In one embodiment, the implanted cells or tissues secrete or produce insulin. In a preferred embodiment, the implanted cells are insulin producing beta-islet cells.

The pouch of the present invention may be comprised of any degradable, bioabsorbable or non-degradable, biocompatible polymer. In a preferred embodiment, the pouch comprises expanded polytetrafluoroethylene (ePTFE). In another preferred embodiment, the pouch comprises polyethyleneterephthalate (PET). In a further embodiment of the present invention, the device is an immunoisolation device, wherein the pouch comprises a semi-permeable or selectively permeable material which permits passage of at least one biological agent, as well as nutrients including, but not limited to, oxygen and glucose, and prevents passage of larger humoral immune molecules and immune cells.

A skilled artisan will appreciate that the subject of the present invention may be any animal, including amphibians, birds, fish, mammals, and marsupials, but is preferably a mammal (*e.g.,* a human; a domestic animal, such as a cat, dog, monkey, mouse, and rat; or a commercial animal, such as a cow, horse or pig). Additionally, the subject of the present invention may be of any age, including a fetus, an embryo, a child, and an adult. In a preferred embodiment of the present invention, the subject is human.

The present invention also provides methods for treating or preventing a disease or disorder in a subject comprising implanting in the subject a device comprising a microvessel construct in contact with a biocompatible, semi-permeable pouch, the pouch encapsulating a cell or cells capable of producing a therapeutically effective amount of a biologically active agent. The vessels of the microvessel construct may be syngenic to the subject, and the implanted cells or tissues may be allogenic or syngenic to the subject. In one embodiment of the invention, the disorder is diabetes, and the implanted cells or tissues secrete or produce insulin. In a preferred embodiment, the disorder is type 1 diabetes or type 2 diabetes, and the implanted cells are insulin secreting cells such as beta-islet cells.

The pouch may be comprised of any degradable, bioabsorbable or non-degradable, biocompatible polymer. In a preferred embodiment, the pouch comprises expanded polytetrafluoroethylene (ePTFE) or polyethyleneterephthalate (PET). In a further embodiment, the device is an immunoisolation device, wherein the pouch comprises a semi-permeable or selectively permeable material which permits passage of at least one biological agent, as well as nutrients including, but not limited to, oxygen and glucose, and prevents passage of larger humoral immune molecules and immune cells.

Also specifically provided are methods for treating or preventing diabetes in a subject comprising implanting in the subject a device comprising a microvessel construct in contact with a biocompatible, semi-permeable pouch, the pouch encapsulating a cell or cells capable of producing a therapeutically effective amount of insulin. In a preferred embodiment, the diabetes is type 1 diabetes or type 2 diabetes, and the insulin producing cells are beta-islet cells.

In addition, it is also described a method for vascularizing an engineered tissue in a subject comprising combining at least one prevascularized construct with the engineered tissue, where the construct contains cells resuspended from a freshly isolated autologous endothelial cell pellet; and then implanting the engineered tissue, thereby vascularizing the engineered tissue *in vivo.* The combining comprises attaching at least one prevascularized construct to the engineered tissue. Attaching may include suturing, stapling, gluing, or combinations thereof. The prevascularized construct may contain cells from at least one endothelial cell pellet obtained from vascular tissue. The vascular tissue can be skin, skeletal muscle, cardiac muscle, atrial appendage of the heart, lung, mesentery, or adipose tissue. Preferably, the vascular endothelial pellet is obtained from a human.

The engineered tissues of the invention may be selected from the group consisting of heart tissue, lung tissue, cardiac muscle tissue, striated muscle tissue, liver tissue, pancreatic tissue, cartilage, bone, pericardium, peritoneum, kidney, smooth muscle, skin, mucosal tissue, small intestine, and large intestine. The engineered tissue can be injected into a subject using, for example, a syringe, needle, cannula, catheter, tube or microneedle.

Also provided are methods for revascularizing a tissue or organ of a subject in need thereof, by injecting into the tissue or organ at least one prevascularized construct containing cells resuspended from a freshly isolated, autologous endothelial cell pellet; and thereby revascularizing the tissue or organ *in vivo.* As previously described above, the vascular tissue may include skin, skeletal muscle, cardiac muscle, atrial appendage of the heart, lung, mesentery, or adipose tissue. Additionally, the adipose tissue may include omental fat, properitoneal fat, perirenal fat, pericardial fat, subcutaneous fat, breast fat, or epididymal fat. The adipose tissue may be obtained by liposuction, abdominoplasty, or combinations thereof.

Further, the organ in need of revascularization may include, without limitation, heart, lung, cardiac muscle, striated muscle, liver, pancreas, kidney, skin, brain, eye, bladder, trachea, diaphragm, ovary, fallopian tube, uterus, small intestine, or large intestine. In another embodiment, the prevascularized construct comprises at least one Relevant Cell, which may be, without limitation, a neuron, myocardiocyte, chondrocyte, pancreatic acinar cell, islet of Langerhans, osteocyte, hepatocyte, Kupffer cell, fibroblast, myocyte, myoblast, satellite cell, adipocyte, preadipocyte, biliary epithelial cell, Purkinje cell, or pacemaker cell. In yet another embodiment, the prevascularized construct may include a cytokine, a chemokine, an antibiotic, a drug, an analgesic agent, an anti-inflammatory agent, an immunosuppressive agent, or any combination thereof.

Methods are also provided for revascularizing a tissue or organ of a subject, by treating the surface of a porous biomaterial with cells from at least one endothelial cell pellet obtained from vascular tissue, wherein the cells are deposited onto the surface of the material and implanted immediately in the subject.

The above-described processes could be incorporated into tissue building methods to establish, prior to implantation, a functional vasculature within a tissue engineered organ or tissue. Characterization of the capillary bed formed in culture and the resulting vasculature present after implantation indicates that the cultured vessels have the potential to differentiate or change into the type of vasculature required to meet specific tissue needs. What this implies is that it may be possible to affect changes to this basic "foundation" microvasculature built in the lab and impart a new character to the microvascular bed to match the type of tissue being built. For example, engineered heart muscle will have a relatively high capillary density, while the vasculature of a liver organoid will exhibit a typical sinusoid-like character. The pre-vascularization process disclosed herein has great potential to incorporate a vascular network within an engineered tissue and engineer it to match a particular tissue of interest, thus overcoming a significant hurdle in tissue engineering.

In tissues suffering from the consequences of chronic ischemic disease, such as after myocardial infarction or peripheral vascular disease, expansion of the vasculature adjacent to the effected tissue areas into the ischemic zones offers one mechanism by which these tissues can be recovered. Implantation of the prevascularized construct could act as a stimulus and nidus for revascularization of the affected areas. In this regard, the implant would act as a nucleus of vascular growth, rapidly establishing a new vascular network within the previously avascular or "hypovascular" zone. The inventors have evidence that the presence of the engineered vessels preserves the surrounding tissue integrity. Insertion of these prevascularized constructs will not only provide for a rapid reperfusion of injured tissues, but may also support the restructuring and repair of those tissues. By incorporating stem cells, progenitor cells or Relevant Cells into the prevascularized construct, cells useful for restructuring, repairing and/or repopulating damaged tissues or organs are provided. In certain embodiments, methods for stimulating or inducing the revascularization of at least one tissue or at least one organ are provided. In certain embodiments, the tissue or organ may be ischemic and/or have a zone or region that is avascular or hypovascular, for example, but not limited to, chronic ischemic disease such as after myocardial infarction, peripheral vascular disease, or cerbrovascular accident (stroke).

Current gene therapy strategies suffer from difficulties in successfully getting the desired gene incorporated into cells of the patient and the therapeutic protein (produced by the recombinant gene) distributed throughout the body. Use of these prevascularized constructs in gene delivery provides 1) a means by which genetically engineered cells included in the tissue construct have ready access to a blood stream (molecular exchange to and from the blood stream occurs best in capillaries) and 2) the culture vessel elements themselves are amenable to genetic engineering and may act as the source of therapeutic gene product. Prevascularized constructs provide a potential means to solving this problem.

In certain embodiments, prevascularized constructs comprising genetically engineered cells are disclosed. Such prevascularized constructs comprising genetically engineered cells are useful in the vascularization and revascularization methods of the invention.

The design and function of the present invention, as well as its advantages, will be more fully appreciated upon reference to the following detailed description having reference to the accompanied drawings.

### BRIEF DESCRIPTION OF THE FIGURES

Many aspects of the disclosure can be better understood with reference to the following drawings which more clearly illustrate the principles of the present disclosure.
FIG. 1 depicts re-vascularization of a microvessel construct. India ink shows patency of transplanted vessels.
FIG. 2 illustrates a hybrid system using a synthetic immunoisolation device (PVID) and a transplanted microcirculation. Arrows indicate the immunoisolation device in contact with the microvessel construct.
FIG. 3 shows a microcirculation formed around a biomaterial that has been treated with a vascular endothelial pellet.
FIG. 4 shows a prevascularized immunoisolation device (PVID). Arrows indicate encapsulated cells, the semi-porous biomaterial, and cells from an endothelial vascular pellet in contact with the semi-porous biomaterial.
FIG. 5 depicts a vascularized construct (sandwich implant) in which islet cells (stained green in micrograph) are positioned or "sandwiched" between the patient's own vascular cells (stained red in micrograph).
FIG. 6 depicts a vascularized construct (sandwich implant) in which islet cells (stained green in micrograph) are positioned or "sandwiched" between a porous biomaterial (ePTFE), and the islet/ePTFE construct is further positioned or "sandwiched" between the patient's own vascular cells.
FIG. 7 depicts insulin staining in control pancreas and in day 7, 14, and 28 sandwich implants. The images demonstrate that detectable quantities of insulin was present at all time points. Additionally, insulin production was seen in intact, as well as partially dissociated islets.

### DETAILED DESCRIPTION OF THE EMBODIMENTS

As used herein and in the appended claims, the singular forms "a," "an," and "the" include plural references unless the content clearly dictates otherwise. Additionally, the section headings used herein are for organizational purposes only and are not to be construed as limiting the subject matter described.

The present invention is based on the discovery that prevascularized devices or constructs, including prevascularized immunoisolation, can improve the efficacy of cell based therapies in a subject by significantly prolonging the viability and function of implanted cells. Importantly, use of the constructs or devices or the invention eliminates the need for immuno-suppressive drugs and their attendant negative side effects. These discoveries have broad implications in the treatment and prevention of diseases and disorders characterized by insufficient levels of one or more particular biologically active agents, including but not necessarily limited to, diabetes.

In one aspect, the present invention presents an implantable device for providing a biologically active agent to a subject, comprising a microvessel construct which is in contact with a biocompatible, semi-permeable pouch, wherein the pouch encapsulates cells or tissues capable of producing the biologically active agent. The invention encompasses microvessel constructs where the vessels of the microvessel construct are syngenic to the subject. The implanted cells or tissues may be allogenic or syngenic to the subject. In one embodiment, the implanted cells or tissues secrete or produce insulin. In a preferred embodiment, the implanted cells are insulin producing beta-islet cells.

In the context of the present invention, the term "biologically active agent" refers to any substance which is capable of exerting a biologically useful effect in the subject. Thus, a biologically active agent encompasses any biologically active molecule, product or solute which provides a metabolic capability or function, such as the removal of specific solutes from the bloodstream; or a biologically active molecule or substance such as, by way of non-limiting example, an enzyme, trophic factor, hormone, neurotransmitter, neuromodulator or biological response modifier.

The pouch of the present invention may be comprised of any degradable, bioabsorbable or non-degradable, biocompatible polymer. In a preferred embodiment, the pouch comprises expanded polytetrafluoroethylene (ePTFE). In another preferred embodiment, the pouch comprises polyethyleneterephthalate (PET). In a further embodiment of the present invention, the device is an immunoisolation device, wherein the pouch comprises a semi-permeable or selectively permeable material which permits passage of at least one biological agent, as well as nutrients including, but not limited to, oxygen and glucose, and prevents passage of larger humoral immune molecules and immune cells.

A skilled artisan will appreciate that the subject of the present invention may be any animal, including amphibians, birds, fish, mammals, and marsupials, but is preferably a mammal (*e.g.,* a human; a domestic animal, such as a cat, dog, monkey, mouse, and rat; or a commercial animal, such as a cow, horse or pig). Additionally, the subject of the present invention may be of any age, including a fetus, an embryo, a child, and an adult. In a preferred embodiment of the present invention, the subject is human.

There are also described methods for treating or preventing a disease or disorder in a subject comprising implanting in the subject a device comprising a microvessel construct in contact with a biocompatible, semi-permeable pouch, the pouch encapsulating a cell or cells capable of producing a therapeutically effective amount of a biologically active agent. The vessels of the microvessel construct may be syngenic to the subject, and the implanted cells or tissues may be allogenic or syngenic to the subject. The disorder may be diabetes, and the implanted cells or tissues secrete or produce insulin. In particular, the disorder is type 1 diabetes or type 2 diabetes, and the implanted cells are insulin secreting cells such as beta-islet cells.

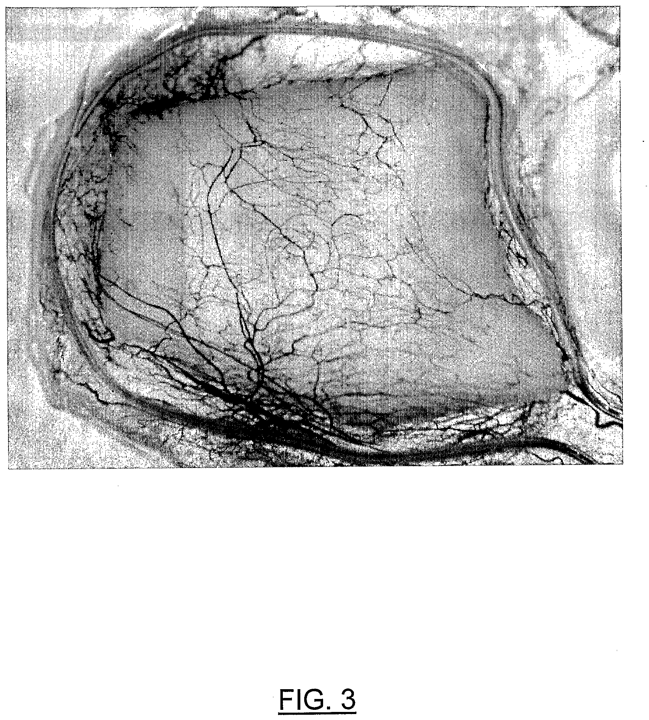

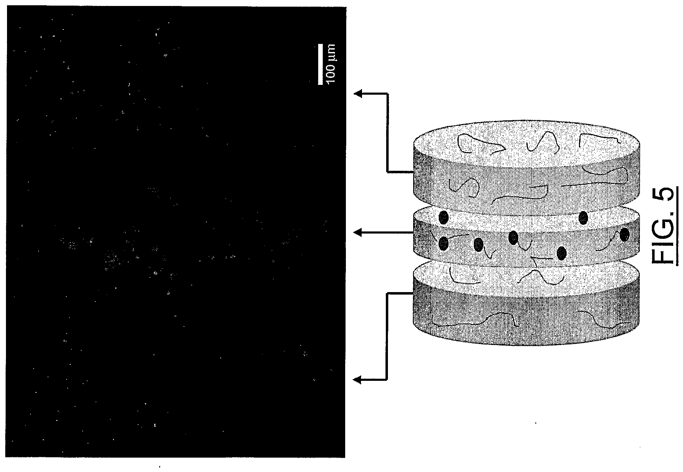

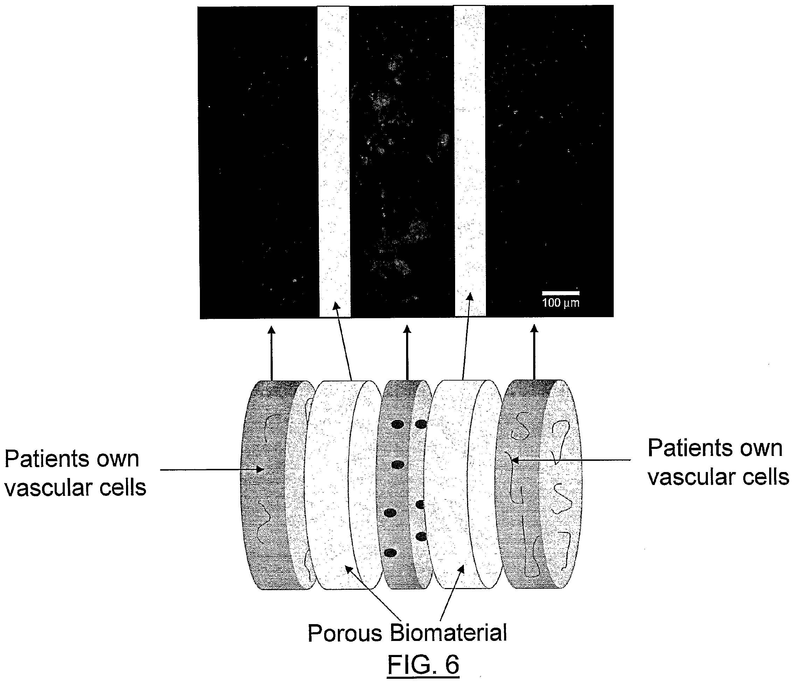

Also provided are method for vascularizing an engineered tissue in a subject comprising combining at least one prevascularized construct with the engineered tissue, where the construct contains cells resuspended from a freshly isolated autologous endothelial cell pellet; and then implanting the engineered tissue, thereby vascularizing the engineered tissue *in vivo.* Fig. 3, for example, shows a microcirculation formed around a biomaterial that has been treated with a vascular endothelial pellet. Combining in the context of the invention may include attaching at least one prevascularized construct to the engineered tissue. Fig. 5 and Fig. 6, for example, depict vascularized "sandwich" constructs in which islet cells are positioned or "sandwiched" between the patients own vascular cells, or alternatively, "sandwiched" between a porous biomaterial, which islet/biomaterial construct may be further sandwiched between the patients own vascular cells.

The term "attaching" may include suturing, stapling, gluing, or other methods known to the skilled artisan or any combination thereof. The prevascularized construct may contain cells from at least one endothelial cell pellet obtained from vascular tissue. The vascular tissue can be skin, skeletal muscle, cardiac muscle, atrial appendage of the heart, lung, mesentery, or adipose tissue. In a preferred embodiment, the vascular endothelial pellet is obtained from a human.

The engineered tissues of the invention may be selected from the group consisting of heart tissue, lung tissue, cardiac muscle tissue, striated muscle tissue, liver tissue, pancreatic tissue, cartilage, bone, pericardium, peritoneum, kidney, smooth muscle, skin, mucosal tissue, small intestine, and large intestine. The engineered tissue can be injected into a subject using, for example, a syringe, needle, cannula, catheter, tube or microneedle.

Also provided are methods for revascularizing a tissue or organ of a subject in need thereof, by injecting into the tissue or organ at least one prevascularized construct containing cells resuspended from a freshly isolated, autologous endothelial cell pellet; and thereby revascularizing the tissue or organ *in vivo.* As previously described above, the vascular tissue may include skin, skeletal muscle, cardiac muscle, atrial appendage of the heart, lung, mesentery, or adipose tissue. Additionally, the adipose tissue may include omental fat, properitoneal fat, perirenal fat, pericardial fat, subcutaneous fat, breast fat, or epididymal fat. In one aspect of the invention, the adipose tissue is obtained by liposuction, abdominoplasty, or combinations thereof.

Further, the organ in need of revascularization may include, without limitation, heart, lung, cardiac muscle, striated muscle, liver, pancreas, kidney, skin, brain, eye, bladder, trachea, diaphragm, ovary, fallopian tube, uterus, small intestine, or large intestine. In another embodiment, the prevascularized construct comprises at least one Relevant Cell, which may be, without limitation, a neuron, myocardiocyte, chondrocyte, pancreatic acinar cell, islet of Langerhans, osteocyte, hepatocyte, Kupffer cell, fibroblast, myocyte, myoblast, satellite cell, adipocyte, preadipocyte, biliary epithelial cell, Purkinje cell, or pacemaker cell. In yet another embodiment, the prevascularized construct may include a cytokine, a chemokine, an antibiotic, a drug, an analgesic agent, an anti-inflammatory agent, an immunosuppressive agent, or any combination thereof.

Methods are also provided for revascularizing a tissue or organ of a subject by treating the surface of a porous biomaterial with cells from one endothelial cell pellet obtained from vascular tissue, wherein the cells are deposited onto the surface of the material and implanted immediately in the subject.

The term "three-dimensional culture" is used in the broad sense herein and refers to a composition comprising a biocompatible matrix, scaffold, or the like. The three-dimensional culture may be liquid, gel, semi-solid, or solid at 25°C. The three-dimensional culture may be biodegradable or non-biodegradable. Exemplary three-dimensional culture materials include polymers and hydrogels comprising collagen, fibrin, chitosan, MATRIGEL, polyethylene glycol, dextrans including chemically crosslinkable or photocrosslinkable dextrans, and the like. In certain embodiments, the three-dimensional culture comprises allogeneic components, autologous components, or both allogeneic components and autologous components. In certain embodiments, the three-dimensional culture comprises synthetic or semi-synthetic materials. In certain embodiments, the three-dimensional culture comprises a framework or support, such as a fibrin-derived scaffold. The term "scaffold" is also used in a broad sense herein. Thus scaffolds include a wide variety of three-dimensional frameworks, for example, but not limited to, a mesh, grid, sponge, foam, or the like.

The terms "engineered tissue", "engineered tissue construct", or "tissue engineered construct" as used herein refer to a tissue or organ that is produced, in whole or in part, using tissue engineering techniques. Descriptions of these techniques can be found in, among other places, "Principles of Tissue Engineering, 2d ed.", Lanza, Langer, and Vacanti, eds., Academic Press, 2000 (hereinafter "Lanza et al."); "Methods of Tissue Engineering", Atala and Lanza, eds., Academic Press, 2001 (hereinafter "Atala et al."); Animal Cell Culture, Masters, ed., Oxford University Press, 2000, (hereinafter "Masters"), particularly Chapter 6; and U.S. Pat. No. 4,963,489 and related U.S. patents.

The term "microvessel fragment" as used herein refers to a segment or piece of vascular tissue, including at least a part or segment of at least one artery, arteriole, capillary, venule, or vein. Typically a microvessel includes endothelial cells arranged in a tube surrounded by one or more layers of mural cells, such as smooth muscle cells or pericytes, and may further comprise extracellular matrix components, such as basement membrane proteins. In certain embodiments, the microvessel fragments are obtained from vascular tissue, for example, but not limited to, skin, skeletal muscle, cardiac muscle, the atrial appendage of the heart, lung, mesentery, or adipose tissue. In certain embodiments, the adipose tissue microvessel fragments are obtained from, for example, but not limited to, subcutaneous fat, perirenal fat, pericardial fat, omental fat, breast fat, epididymal fat, properitoneal fat, and the like. The skilled artisan will appreciate that other fat deposits or any vascular-rich tissue or organ may serve as a source of microvessel fragments for use in the invention, for example, but not limited to, skin, muscle, including skeletal or cardiac muscle, lung, and mesentery. In certain embodiments, the microvessel fragments are obtained from adipose tissue harvested by liposuction or abdominoplasty. Adipiose tissue harvested by a liposuction procedure where a sonic probe is not used during the harvesting process is particularly useful.

The term "endothelial cell pellet" as used in the context of the present invention refers to a mass of endothelial cells, e.g., vascular endothelial cells, prepared according to any of the cell pellet formation methods well known to the skilled artisan. In certain embodiments. Typically a microvessel includes endothelial cells arranged in a tube surrounded by one or more layers of mural cells, such as smooth muscle cells or pericytes, and may further comprise extracellular matrix components, such as basement membrane proteins. In certain embodiments, the endothelial cell pellets are obtained from vascular tissue, for example, but not limited to, skin, skeletal muscle, cardiac muscle, the atrial appendage of the heart, lung, mesentery, or adipose tissue. In certain embodiments, the adipose tissue endothelial cell pellets are obtained from, for example, but not limited to, subcutaneous fat, perirenal fat, pericardial fat, omental fat, breast fat, epididymal fat, properitoneal fat, and the like. The skilled artisan will appreciate that other fat deposits or any vascular-rich tissue or organ may serve as a source of endothelial cell pellets for use in the invention, for example, but not limited to, skin, muscle, including skeletal or cardiac muscle, lung, and mesentery. In certain embodiments, the endothelial cell pellets are obtained from adipose tissue harvested by liposuction or abdominoplasty. Adipiose tissue harvested by a liposuction procedure where a sonic probe is not used during the harvesting process is particularly useful.

The terms "vascularize", "vascularizing", or "vascularization" as used herein refer to providing a functional or substantially functional vascular network to an organ or tissue, particularly an engineered tissue. A functional or substantially functional vascular network is one that perfuses or is capable of perfusing the tissue or organ to meet some or all of the tissue's or organ's nutritional needs, oxygen demand, and waste product elimination needs. A vascular tissue is a natural tissue that is rich in vascular elements, such as microvessels, for example, but without limitation, adipose tissue.

The terms "revascularize", "revascularizing", "neovascularization", or "revascularization" as used herein refer to revising an existing vascular network or establishing a new functional or substantially functional vascular network in a tissue or organ that has an avascular or hypovascular zone, typically due to disease, congenital defect, or injury. Additionally, the topical application of certain chemotherapeutic agents, for example, but not limited to, 5-flourouracil (5-FU), may also result in an ischemic or avascular zone. Such an avascular or hypovascular tissue or organ is often totally or partially dysfunctional or has limited function and may be in need of revascularization. Revascularizing such a tissue or organ may result in restored or augmented function.

As used herein, the term "polymer" is used in the broad sense and is intended to include a wide range of biocompatible polymers, for example, but not limited to, homopolymers, co-polymers, block polymers, cross-linkable or crosslinked polymers, photoinitiated polymers, chemically initiated polymers, biodegradable polymers, nonbiodegradable polymers, and the like. In other embodiments, the prevascularized construct comprises a polymer matrix that is nonpolymerized, to allow it to be combined with a tissue, organ, or engineered tissue in a liquid or semi-liquid state, for example, by injection. In certain embodiments, the prevascularized construct comprising liquid matrix may polymerize or substantially polymerize *"in vitro."* In certain embodiments, the prevascularized construct is polymerized or substantially polymerized prior to injection. Such injectable compositions are prepared using conventional materials and methods know in the art, including, but not limited to, Knapp et al., Plastic and Reconstr. Surg. 60:389 405, 1977; Fagien, Plastic and Reconstr. Surg. 105:362 73 and 2526 28, 2000; Klein et al., J. Dermatol. Surg. Oncol. 10:519 22, 1984; Klein, J. Amer. Acad. Dermatol. 9:224 28, 1983; Watson et al., Cutis 31:543 46, 1983; Klein, Dermatol. Clin. 19:491 508, 2001; Klein, Pedriat. Dent. 21:449 50, 1999; Skorman, J. Foot Surg. 26:511 5, 1987; Burgess, Facial Plast. Surg. 8:176 82, 1992; Laude et al., J. Biomech. Eng. 122:231 35, 2000; Frey et al., J. Urol. 154:812 15, 1995; Rosenblatt et al., Biomaterials 15:985 95, 1994; Griffey et al., J. Biomed. Mater. Res. 58:10 15, 2001; Stenburg et al., Scfand. J. Urol. Nephrol. 33:355 61,1999; Sclafani et al., Facial Plast. Surg. 16:29 34, 2000; Spira et al., Clin. Plast. Surg. 20:181 88, 1993; Ellis et al., Facila Plast. Surg. Clin. North Amer. 9:405 11, 2001; Alster et al., Plastic Reconstr. Surg. 105:2515 28, 2000; and U.S. Pat. Nos. 3,949,073 and 5,709,854.

In certain embodiments, the polymerized or nonpolymerized matrix comprises collagen, including contracted and non-contracted collagen gels, hydrogels comprising, for example, but not limited to, fibrin, alginate, agarose, gelatin, hyaluronate, polyethylene glycol (PEG), dextrans, including dextrans that are suitable for chemical crosslinking, photocrosslinking, or both, albumin, polyacrylamide, polyglycolyic acid, polyvinyl chloride, polyvinyl alcohol, poly(n-vinyl-2-pyrollidone), poly(2-hydroxy ethyl methacrylate), hydrophilic polyurethanes, acrylic derivatives, pluronics, such as polypropylene oxide and polyethylene oxide copolymer, or the like. In certain embodiments, the fibrin or collagen is autologous or allogeneic with respect to the intended recipient. The skilled artisan will appreciate that the matrix may comprise non-degradable materials, for example, but not limited to, expanded polytetrafluoroethylene (ePTFE), polytetrafluoroethylene (PTFE), polyethyleneterephthalate (PET), polyurethane, polyethylene, polycarbonate, polystyrene, silicone, and the like, or selectively degradable materials, such as poly (lactic-co-glycolic acid; PLGA), PLA, or PGA. (See also, Middleton et al., Biomaterials 21:2335 2346, 2000; Middleton et al., Medical Plastics and Biomaterials, March/April 1998, at pages 30 37; Handbook of Biodegradable Polymers, Domb, Kost, and Domb, eds., 1997, Harwood Academic Publishers, Australia; Rogalla, Minim. Invasive Surg. Nurs. 11:67 69, 1997; Klein, Facial Plast. Surg. Clin. North Amer. 9:205 18, 2001; Klein et al., J. Dermatol. Surg. Oncol. 11:337 39, 1985; Frey et al., J. Urol. 154:812 15, 1995; Peters et al., J. Biomed. Mater. Res. 43:422 27, 1998; and Kuijpers et al., J. Biomed. Mater. Res. 51:136 45, 2000).

### I. Prevascularized Constructs.

The terms "prevascularized construct" or "engineered microvascular network" refer to a composition comprising at least one microvessel fragment, or cells from an endothelial cell pellet, typically isolated from a vascular-rich tissue, in a three-dimensional culture, including but not limited to, a matrix, scaffold, gel, or liquid. In certain embodiments, the prevascularized constructs comprise a three-dimensional matrix and microvessel fragments. In certain embodiments, the matrix comprises a preformed framework, for example, but not limited to, a fibrin scaffold. In certain embodiments, the three-dimensional culture comprises a polymerized, substantially polymerized, or nonpolymerized matrix.

In certain embodiments, prevascularized constructs are prepared by combining microvessel fragments or cells from an endothelial cell pellet, and a liquid three-dimensional culture, such as nonpolymerized collagen, agarose, gelatin, other nonpolymerized polymer matrices, or the like. In other embodiments, the microvessel fragments or cells from an endothelial cell pellet, are seeded, sodded or perfused onto or through a solid or semi-solid three-dimensional culture environment, for example, but not limited to, a framework, scaffold, hollow-fiber filter, or the like.

Prevascularized constructs may be categorized as "cultured microvessel constructs" or "freshly isolated microvessel constructs." A cultured microvessel construct is typically incubated prior to implantation. For example, but not limited to, in a humidified incubator at 37°C. and 5% CO₂. Typically such cultured microvessel constructs are incubated for a period of one hour to thirty days, but may be incubated for shorter or longer periods, as desired. The skilled artisan will appreciate that the term "cultured" may or may not refer to the use of conventional incubation methods, such as a controlled-temperature incubator. Alternately, a prevascularized construct may comprise a freshly isolated microvessel construct that undergoes little or no incubation prior to use. The skilled artisan will appreciate that freshly isolated microvessel constructs may, but need not, be incubated. In certain embodiments, a freshly isolated microvessel construct comprises microvessel fragments, or cells from a vascular endothelial pellet, in a three-dimensional culture that has been "incubated" subsequent to the introduction of the microvessels, for example, but without limitation, to allow the construct to polymerize. In other embodiments, a freshly isolated microvessel construct comprises a liquid three-dimensional culture, as may be appropriate for implantation by injection (see, e.g., U.S. Pat. Nos. 5,709,854 and 6,224,893). Such liquid constructs may, but need not, polymerize *in vitro* under appropriate conditions.

The skilled artisan will understand that prevascularized constructs comprising a nonpolymerized liquid three-dimensional culture that is subsequently allowed to polymerize or gel are capable of assuming a multitude of shapes. Thus, in certain embodiments, the ultimate size and shape of the polymerized construct depends, in part, on the size and shape of the vessel in which the construct is polymerized. For example, but not limited to, cylindrical or tubular constructs can be prepared using conical tubes; disk-shaped constructs can be prepared using multi-well plates; planar constructs can be prepared using flat surfaces, for example, a petri dish, the inverted lid of a multi-well plate, or a flat-bottomed dish. Additionally, in certain embodiments, polymerized prevascularized constructs can be cut or trimmed into a desired size or shape. Thus, prevascularized constructs can be prepared in virtually any size and shape, prior to or during use.

In certain embodiments, the prevascularized construct comprises autologous microvessel fragments or cells from an endothelial cell pellet, in an autologous or substantially autologous three-dimensional culture. In certain embodiments, prevascularized constructs comprise microvessel fragments in a three-dimensional culture comprising a scaffold, for example, but not limited to, fibrin-derived scaffolds (see, e.g., Nicosia et al., Lab. Invest. 63:115 22, 1990) and scaffolds comprising artificial, FDA-approved synthetic biocompatible polymers, for example, but not limited to, polyethylene, polymethacrylate, polyurethane, vinyl, such as polyvinyl chloride, silicones, PLGA, PTFE, ePTFE, polypropylene, polyethyleneterephthalate (PET), nylon, polylactide, and polyglycolide. Discussions of exemplary biocompatible polymers, scaffolds, and other matrix materials, including protocols for their preparation and use, may be found in, among other places, Atala et al., particularly Chapters 42 76; Lanza et al., particularly Chapters 21 and 22; and Handbook of Biodegradable Polymers, Domb, Kost, and Domb, eds., 1997, Harwood Academic Publishers, Australia.

In certain embodiments, the prevascularized constructs comprise microvessel fragments that are autologous or allogeneic with respect to the intended human or animal recipient. In certain embodiments, the prevascularized construct further comprises at least one cytokine, at least one chemokine, at least one antibiotic, such as an antimicrobial agent, at least one drug, at least one analgesic agent, at least one anti-inflammatory agent, at least one immunosuppressive agent, or various combinations thereof. In certain embodiments, the at least one cytokine, at least one antibiotic, at least one drug, at least one analgesic agent, at least one anti-inflammatory agent, at least one immunosuppressive agent, or various combinations thereof comprise a controlled-release format, such as those generally known in the art, for example, but not limited to, Richardson et al., Nat. Biotechnol. 19:1029 34, 2001.

Exemplary cytokines include angiogenin, vascular endothelial growth factor (VEGF, including, but not limited to VEGF-165), interleukins, fibroblast growth factors, for example, but not limited to, FGF-1 and FGF-2, hepatocyte growth factor, (HGF), transforming growth factor beta (TGF-.beta.), endothelins (such as ET-1, ET-2, and ET-3), insulin-like growth factor (IGF-1), angiopoietins (such as Ang-1, Ang-2, Ang-3/4), angiopoietin-like proteins (such as ANGPTL1, ANGPTL-2, ANGPTL-3, and ANGPTL-4), platelet-derived growth factor (PDGF), including, but not limited to, PDGF-AA, PDGF-BB and PDGF-AB, epidermal growth factor (EGF), endothelial cell growth factor (ECGF), including ECGS, platelet-derived endothelial cell growth factor (PD-ECGF), placenta growth factor (PLGF), and the like. Cytokines, including recombinant cytokines, and chemokines are typically commercially available from numerous sources, for example, R & D Systems (Minneapolis, Minn.); Endogen (Woburn, Wash.); and Sigma (St. Louis, Mo.). The skilled artisan will understand that the choice of chemokines and cytokines for incorporation into particular prevascularized constructs will depend, in part, on the target tissue or organ to be vascularized or revascularized.

In certain embodiments, prevascularized constructs further comprise at least one genetically engineered cell. In certain embodiments, prevascularized constructs comprising at least one genetically engineered cell will constitutively express or inducibly express at least one gene product encoded by the at least one genetically engineered cell due to the genetic alterations within at least one genetically engineered cell induced by techniques known in the art. Descriptions of exemplary genetic engineering techniques can be found in, among other places, Ausubel et al., Current Protocols in Molecular Biology (including supplements through March 2002), John Wiley & Sons, New York, N.Y., 1989; Sambrook et al., Molecular Cloning: A Laboratory Manual, 2.sup.nd Ed., Cold Spring Harbor Laboratory Press, Cold Spring Harbor, N.Y., 1989; Sambrook and Russell, Molecular Cloning: A Laboratory Manual, 3.sup.rd Ed., Cold Spring Harbor Laboratory Press, Cold Spring Harbor, N.Y., 2001; Beaucage et al., Current Protocols in Nucleic Acid Chemistry, John Wiley & Sons, New York, N.Y., 2000 (including supplements through March 2002); Short Protocols in Molecular Biology, 4.sup.th Ed., Ausbel, Brent, and Moore, eds., John Wiley & Sons, New York, N.Y., 1999; Davis et al., Basic Methods in Molecular Biology, McGraw Hill Professional Publishing, 1995; Molecular Biology Protocols (see the highveld.com website), and Protocol Online (protocol-online.net). Exemplary gene products for genetically modifying the genetically engineered cells of the invention include plasminogen activator, soluble CD4, Factor VIII, Factor IX, von Willebrand Factor, urokinase, hirudin, interferons, including alpha-, beta- and gamma-interferon, tumor necrosis factor, interleukins, hematopoietic growth factor, antibodies, glucocerebrosidase, adenosine deaminase, phenylalanine hydroxylase, human growth hormone, insulin, erythropoietin, VEGF, angiopoietin, hepatocyte growth factor, PLGF, and the like.

In certain embodiments, the prevascularized construct further comprises appropriate stromal cells, stem cells, Relevant Cells, or combinations thereof. As used herein, the term "stem cells" is used in a broad sense and includes traditional stem cells, progenitor cells, preprogenitor cells, reserve cells, and the like. Exemplary stem cells include embryonic stem cells, adult stem cells, pluripotent stem cells, neural stem cells, liver stem cells, muscle stem cells, muscle precursor stem cells, endothelial progenitor cells, bone marrow stem cells, chondrogenic stem cells, lymphoid stem cells, mesenchymal stem cells, hematopoietic stem cells, central nervous system stem cells, peripheral nervous system stem cells, and the like. Descriptions of stem cells, including method for isolating and culturing them, may be found in, among other places, Embryonic Stem Cells, Methods and Protocols, Turksen, ed., Humana Press, 2002; Weisman et al., Annu. Rev. Cell. Dev. Biol. 17:387 403; Pittinger et al., Science, 284:143 47, 1999; Animal Cell Culture, Masters, ed., Oxford University Press, 2000; Jackson et al., PNAS 96 (Shepherd BR et al. Rapid perfusion and network remodeling in a microvascular construct after implantation. Arterioscler Thromb Vasc Biol 24: 898-904, 2004):14482 86, 1999; Zuk et al., Tissue Engineering, 7:211 228, 2001 ("Zuk et al."); Atala et al., particularly Chapters 33 41; and U.S. Pat. Nos. 5,559,022, 5,672,346 and 5,827,735. Descriptions of stromal cells, including methods for isolating them, may be found in, among other places, Prockop, Science, 276:71 74, 1997; Theise et al., Hepatology, 31:235 40, 2000; Current Protocols in Cell Biology, Bonifacino et al., eds., John Wiley & Sons, 2000 (including updates through March, 2002); and U.S. Pat. No. 4,963,489. The skilled artisan will understand that the stem cells and/or stromal cells selected for inclusion in a prevascularized construct are typically appropriate for the intended use of that construct.

The term "Relevant Cells", as used herein refers to cells that are appropriate for incorporation into a prevascularized construct, based on the intended use of that construct. For example, Relevant Cells that are appropriate for the repair, restructuring, or repopulation of damaged liver may include, without limitation, hepatocytes, biliary epithelial cells, Kupffer cells, fibroblasts, and the like. Exemplary Relevant Cells for incorporation into prevascularized constructs include neurons, myocardiocytes, myocytes, chondrocytes, pancreatic acinar cells, islets of Langerhans, osteocytes, hepatocytes, Kupffer cells, fibroblasts, myocytes, myoblasts, satellite cells, endothelial cells, adipocytes, preadipocytes, biliary epithelial cells, and the like. These types of cells may be isolated and cultured by conventional techniques known in the art. Exemplary techniques can be found in, among other places, Atala et al., particularly Chapters 9 32; Freshney, Culture of Animal Cells A Manual of Basic Techniques, 4th ed., Wiley Liss, John Wiley & Sons, 2000; Basic Cell Culture: A Practical Approach, Davis, ed., Oxford University Press, 2002; Animal Cell Culture: A Practical Approach, Masters, ed., 2000; and U.S. Pat. Nos. 5,516,681 and 5,559,022.

The skilled artisan will appreciate that such stromal cells, stem cells, and/or Relevant Cells may be incorporated into the prevascularized constructs during or after preparation. For example, but not limited to, combining microvessel fragments, stem cells, Relevant Cells, and/or stromal cells in a liquid three-dimensional culture, such as collagen, fibrin, or the like, or seeding or sodding stem cells, Relevant Cells, and/or stromal cells in or on a prevascularized construct may be achieved. Exemplary combinations of appropriate stem cells, stromal cells, and Relevant Cells for incorporation into prevascularized constructs include: islets of Langerhans and/or pancreatic acinar cells in a prevascularized construct for revascularizing a damaged pancreas; hepatocytes, hepatic progenitor cells, Kupffer cells, endothelial cells, endodermal stem cells, liver fibroblasts, and/or liver reserve cells in a prevascularized construct for revascularizing a damaged liver. For example, but not limited to, appropriate stem cells or stromal cells for a prevascularized construct for vascularizing, repairing, and reconstructing a damaged or disease liver might comprise liver reserve cells, liver progenitor cells, such as, but not limited to, liver fibroblasts, embryonic stem cells, liver stem cells; cardiomyocytes, Purkinje cells, pacemaker cells, myoblasts, mesenchymal stem cells, satellite cells, and/or bone marrow stem cells for revascularizing a damaged or ischemic heart (see, e.g., Atkins et al., J. of Heart and Lung Transplantation, December 1999, at pages 1173 80; Tomita et al., Cardiovascular Research Institute, American Heart Association, 1999, at pages 92 101; Sakai et al., Cardiovascular Research Institute, American Heart Association, 1999, at pages 108 14), and the like.

### II. Methods for Vascularizing Engineered Tissues and Organs

Methods are provided for vascularizing engineered tissues comprising combining at least one prevascularized construct with an engineered tissue to produce a vascularized engineered tissue. In certain embodiments, prevascularized constructs for vascularizing engineered tissues further comprise at least one stromal, stem cell, Relevant Cell, or genetically engineered cell. In certain embodiments, prevascularized construct for vascularizing engineered tissues comprise at least one cytokine, chemokine, antibiotic, drug, analgesic, anti-inflammatory, or the like. Methods for preparing engineered tissues are well known in the art. Descriptions of such techniques may be found in, among other places, Atala et al.; Lanza et al.; Masters; and in U.S. Pat. Nos. 4,963,489; 5,266,480; 5,510,254; 5,512,475; 5,516,680; 5,516,681; 5,518,915; 5,541,107; 5,578,485; 5,624,840; 5,763,267; 5,785,964; 5,792,603; 5,842,477; 5,858,721; 5,863,531; 5,902,741; 5,962,325; 6,022,743; 6,060,306; 6,121,042; and 6,218,182.

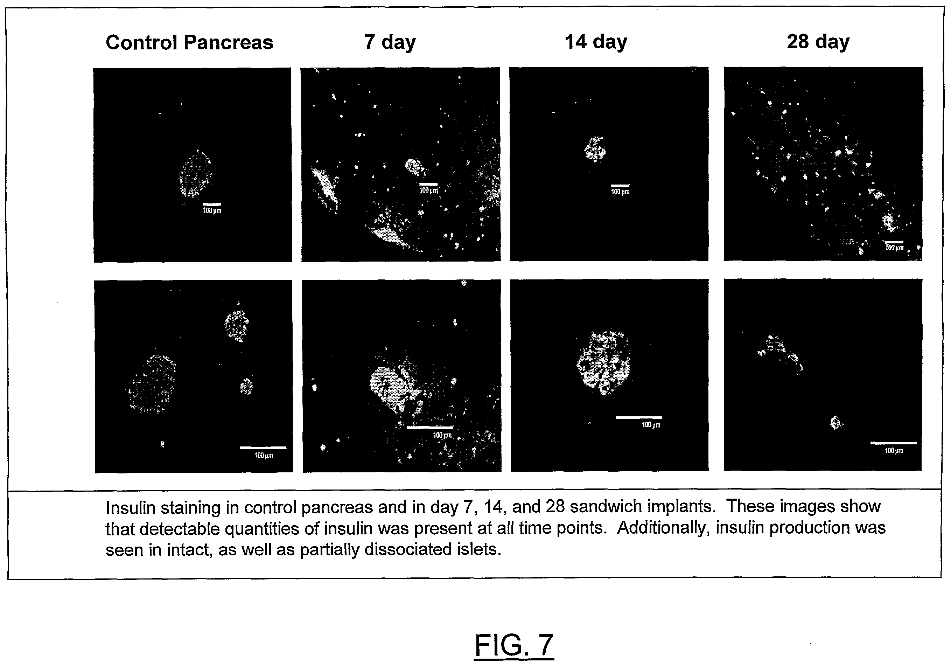

According to certain methods for vascularizing engineered tissues, the term "combining" comprises placing or implanting at least one prevascularized construct on any surface of, within, between layers of, or adjacent to, the engineered tissue. Fig. 6 and Fig. 7, for example, depict vascularized "sandwich" constructs in which islet cells are positioned or "sandwiched" between the patients own vascular cells, or alternatively, "sandwiched" between a porous biomaterial, which islet/biomaterial construct may be further sandwiched between the patients own vascular cells. Combining may comprise coating the engineered tissue with a prevascularized construct. For example, but without limitation, an engineered tissue is dipped into a liquid prevascularized construct or a liquid prevascularized construct is poured or sprayed on an engineered tissue. Such liquid prevascularized construct coating the engineered tissue may be polymerized. Such coated engineered tissues may be incubated prior to implantation into a recipient animal or human. In certain embodiments, the prevascularized construct is combined with the engineered tissue by injection.

The terms "injecting", "injection", or variations thereof as used herein shall refer to any means of ejecting or extruding a substance, typically through a tube or structure comprising a bore or external opening. Such tube or structure can be flexible, inflexible, or can comprise at least one flexible portion and at least one inflexible portion. Exemplary injection means include a syringe with or without a needle, a cannula, a catheter, flexible tubing, and the like. Delivery of a prevascularized construct might also be accomplished through the use of devices that permeablize tissue, such as microneedles. In contrast to traditional injections with standard-gauge hypodermic needles, microneedle (typically defined by a radius of curvature .about.1 um) or microneedle arrays permeabilize the skin or endothelial cell layer by producing microscopic holes. These holes, in effect, act as conduits for materials delivery and may enhance the attachment or delivery of a prevascularized construct to a vessel, tissue, or organ. Thus, the skilled artisan will understand that any structure comprising a bore or external opening through which at least one prevascularized construct can be extruded on or into a tissue or organ, or any structure that can permeabilize the surface of a tissue or and organ, including an engineered tissue, is within the intended scope of the invention. In certain embodiments, such injected construct polymerizes *in vitro,* following injection. In certain embodiments, such injected prevascularized construct comprises at least one cultured microvessel construct, at least one freshly isolated microvessel construct, or both.

In certain embodiments, combining at least one prevascularized construct with an engineered tissue comprises attaching at least one prevascularized construct to at least one engineered tissue, using techniques known in the art. Exemplary attachment means include suturing, stapling, for example, with surgical staples, glue or adhesive, such as surgical glue, biochemical interactions such as with the extracellular matrix, photo-activated glue, fibrin glue, acrylate-based adhesives, and the like.

In certain embodiments, combining comprises placing the at least one prevascularized construct between the layers of an engineered tissue, such that at least one surface of at least one prevascularized construct is adjacent to, or in contact with, at least one surface of at least one engineered tissue. In certain embodiments, combining comprises inserting or implanting at least one prevascularized construct within an engineered tissue, for example, but not limited to, within a designed pocket, bore, crevice, or the like. In certain embodiments, the prevascularized construct is inserted within an incision in the engineered tissue. In certain embodiments, combining comprises wrapping at least one prevascularized construct around or within at least one engineered tissue, such that the prevascularized construct envelopes or substantially envelopes the engineered tissue, or is enveloped or substantially enveloped by the engineered tissue. In certain embodiments, combining comprises forming or incorporating at least one prevascularized construct into the engineered tissue during the tissue engineering process. In certain embodiments, combining comprises culturing at least one prevascularized construct on or within a growing engineered tissue during the tissue engineering process, such as in a bioreactor. In certain embodiments, at least one prevascularized construct is enveloped or substantially enveloped by the adjacent tissue or organ during the tissue engineering process.

In certain embodiments, the combined engineered tissue and at least one prevascularized construct are incubated, for example, within a bioreactor or humidified incubator, prior to *in vivo* implantation into a recipient animal or human. In certain embodiments, combining comprises implanting at least one engineered tissue comprising at least one prevascularized construct directly into a recipient animal or human with little or no additional incubation.

In certain embodiments, the implanted prevascularized construct serves as a nucleation site for vascularizing the engineered tissue. In certain embodiments, appropriate stromal cells, stem cells, and/or Relevant Cells from the prevascularized construct will support the integration of the engineered tissue within the recipient animal or human. Constructs comprising genetically engineered cells may produce recombinant products that are distributed systemically via the bloodstream or delivered to the local microenvironment to induce repair, wound healing, or the like.

### III. Methods for Revascularizing Damaged or Injured Tissues or Organs

Methods are provided for revascularizing damaged or injured tissues or organs, i.e., tissues or organs in need of revascularization and repair or reconstruction, are provided. In certain embodiments, prevascularized constructs for revascularizing tissues or organs further comprise at least one appropriate stromal cell, stem cell, Relevant Cell, or genetically engineered cell. In certain embodiments, prevascularized constructs for revascularizing tissues or organs comprise at least one cytokine, chemokine, antibiotic, drug, analgesic, anti-inflammatory, or the like. In certain embodiments, the prevascularized construct, once implanted *in vivo,* will develop a functional vascular bed and inosculate with the surrounding functional vascular system and perfuse, or be capable of perfusing, the damaged tissue or organ.

According to certain methods for revascularizing tissues or organs, at least one prevascularized construct is combined with the tissue or organ and a revascularized tissue or organ is generated. According to certain methods for revascularizing tissues or organs, the term "combining" comprises placing or implanting at least one prevascularized construct on any surface of, within, between the layers of, or adjacent to, the tissue or organ. In certain embodiment, the prevascularized construct is implanted in the tissue or organ by injection. In certain embodiments, such injected construct will polymerize *in vitro,* following implantation. In certain embodiments, such injected prevascularized construct comprises at least one cultured microvessel construct, at least one freshly isolated microvessel construct, or both. In certain embodiments, combining comprises attaching at least one prevascularized construct to at least one tissue or organ in need of revascularizing, using techniques known in the art, such as described above.

The skilled artisan understands that certain tissues and organs are covered by or contain a layer of fibrous tissue, connective tissue, fatty tissue, or the like, and that the underlying tissue or organ can be revascularized without removing this layer. Such a layer may be naturally occurring (such as a serosal layer, mucous membrane, fibrous capsule, or the like), may result form fibrosis, necrosis, or ischemia, due to disease, defect, injury, or biochemical deficiency. Typically, the microvessel fragments of the prevascularized construct can penetrate such a layer and inosculate with the vasculature of the underlying tissue or organ, revascularizing the tissue or organ. Thus, combining the prevascularized construct with the tissue or organ in need of revascularization, comprises placing the prevascularized construct on or in such layer. For example, but not limited to, placing the prevascularized construct on the meninges to revascularize brain tissue; the epicardium to revascularize the myocardium; the peritoneum and/or serosa, to revascularize portions of the large intestine; the conjunctiva and/or subconjunctiva to revascularize the eye; the tracheal surface to revascularize the trachea; the bucchal mucosa to revascularize the mouth; the pleural and/or serosal surface to revascularize the lung; the pleural and/or peritoneal surface to revascularize the diaphragm; the skin to revascularize non-healing skin ulcers, such as diabetic ulcers; the pericardial surface to revascularize the pericardium; and the like.

In certain embodiments, the prevascularized construct, when combined with the tissue or organ within the animal or human, will develop functional vascular bed and inosculate with the surrounding functional vascular system and perfuse the damaged tissue or organ. In certain embodiments, the implanted prevascularized construct serves as a nucleation site for revascularizing the damaged tissue or organ. In certain embodiments, appropriate stem cells, stromal cells, and/or Relevant Cells from the prevascularized construct will support the restructuring and repair of the damaged tissue or organ. Constructs comprising genetically engineered cells may produce recombinant products that are distributed systemically via the bloodstream or delivered to the local microenvironment to induce repair, wound healing, or the like.

The invention, having been described above, may be better understood by reference to examples. The following examples are intended for illustration purposes only, and should not be construed as limiting the scope of the invention in any way.

### EXAMPLES

### Summary

A medical technology for islet cell transplantation that avoids the use of immunosuppressive drugs remains a major goal to correct diabetic hyperglycemia. These experiments evaluate a novel immunoisolation device that is constructed using a preformed microcirculation. The ability to maintain islet cell viability *in vivo* using this prevascularized construct is explored. The cell transplantation methods explored have compatibility with the human operating room and the feasibility of translating these preclinical studies to the human clinical setting for treatment of diabetes is evaluated.

### Evaluation of PVID

Implantation of any device or tissue is at risk for early failure due to the time necessary to recruit and establish a stable vasculature. The inventors have developed methods to pre-form an adaptive microvascular system capable of rapidly progressing into a mature, efficient perfusion circuit upon implantation. Using this system, a device for use with islet transplantation is prevascularized in order to demonstrated that prevascularizing an immunoisolation construct accelerates the formation of a stable microcirculation around the construct following implantation.

The prevascularized construct is also evaluated for its ability to support long term islet function following transplantation. Specifically, the ability of PVIDs to provide nutrients to islets placed into immunoisolation devices is evaluated in order to demonstrate that the accelerated formation of a microcirculation around the immunoisolation device will extend islet cell function.

### Immunoisolation Devices and the Microcirculation

A critical need in allograft islet cell transplantation is the isolation of this allograft material from immune system recognition and subsequent destruction. A variety of immunoisolation devices are under development which provide possible solutions to the immune response issue (Berney T. and Ricordi C. Immunoisolation of cells and tissues for transplantation. Cell Transplantation 8: 577-579, 1999). The use of synthetic implants with selective permeability has been reported with respect to the development of immunoisolation devices. Brauker has reported that polymers of specific design can be manufactured that stimulate an angiogenic response, resulting in a polymer with commercial value as an immunoisolation device (Brauker J.H. et al. Neovascularization of synthetic membranes directed by membrane microarchitecture. Journal of Biomedical Materials Research 29: 1517-1524, 1995). The ability to form and maintain a functional microcirculation on the external surface of these devices is paramount to their eventual biological success since they must provide nutrients to the inner tissue, remove synthetic cellular products, and maintain a barrier to cellular immune response. The inventors will directly address a significant shortcoming in current approaches using implanted islets to overcome hyperglycemia. The inventors' strategy involves the integration of existing, novel technologies to uniquely accommodate islet transplants and construct an islet transplant with long term viability and function. The most critical problem to address is the formation of a stable microcirculation in association with these implanted immunoisolation devices.

### Microvascular Homeostasis

Within most mature tissues the microcirculation has reached an equilibrium or homeostasis wherein new blood vessels are neither increasing or decreasing in density. The capillary endothelium exhibits morphological characteristics specific to the function of the tissue perfused. For example, capillaries perfusing pancreatic islets exhibit fenestrations in their membranes, liver endothelium exhibits gaps between cells to permit rapid cellular exchange and brain endothelium exhibits tight junctions between cells establishing the blood-brain barrier. This homeostatis in vessel growth is regulated by numerous cell types and metabolic factors. This balance can be altered resulting in both a stimulation of new vessel development; a process generally referred to as angiogenesis, and a stimulation of vessel degeneration or regression. Would healing is one example wherein microvascular homeostasis or "angiostasis" is disrupted often resulting in a period of angiogenesis. The temporal events of wound healing often include a period of "angioregression" resulting in, for example, granulation tissue that contains predominantly capillaries in limited number.

In the field of biomaterial implants and peri-implant healing responses, the development of an vascular fibrous capsule provides the most germane example of the elements of angiostasis, angiogenesis and angioregression (Auerbach R. et al. Angiogenesis assays: a critical overview. Clin Chem 49: 32-40, 2003; Djonov V. et al. Vascular remodeling by intussusceptive angiogenesis. Cell Tissue Res 2003; Folkman J. Anti-angiogenesis: new concept for therapy of solid tumors. Am Surg 175: 409-416, 1972; Folkman J. Tumor angiogenesis. Adv Cancer Res 19: 331-358, 1974; Hoying J.B. et al. Heterogeneity in Angiogenesis. In: Genetics of Angiogenesis, edited by Hoying J.B. BIOS Scientific Publishers Ltd., 2003, p. 191-203; Ingber D.E. Mechanical signaling and the cellular response to extracellular matrix in angiogenesis and cardiovascular physiology. Circ Res 91: 877-887; 2002; Kale S. et al. Microarray analysis of in vitro pericyte differentiation reveals an angiogenic program of gene expression. FASEB J 19: 270-271; 2005; Pierce S. and Skalak T.C. Microvascular remodeling: a complex continuum spanning angiogenesis to arteriogenesis. Microcirculation 10: 99-111, 2003; Rifkin D.B. et al. The involvement of proteases and protease inhibitors in neovascularization. Acta Biol Med Ger 40: 1259-1263, 1981; Wahlberg E. Angiogenesis and arteriogenesis in limb ischemia. J Vasc Surg 38: 198-203, 2003). This homeostasis will be altered toward the development of a mature fully functional microcirculation in association with implanted materials and devices.

The mechanisms underlying the formation of a microcirculation during development (vasculogenesis) and from an existing vasculature (angiogenesis) are under intense evaluation by numerous investigators (Ashley RA et al. Erythropoietin stimulates vasculogenesis in neonatal rat mesenteric microvascular endothelial cells. Pediatr Res 51: 472-478, 2002; Flamme I et al. Vascular endothelial growth factor (VEGF) and VEGF receptor 2 (flk-1) are expressed during vasculogenesis and vascular differentiation in the quail embryo. Dev Biol 169: 699-712, 1995; Risau W and Flamme I. Vasculogenesis. [Review] [122 refs]. Annual Review of Cell & Developmental Biology 11: 73-91, 1995; Shalaby F. et al. Failure of blood-island formation and vasculogenesis in Flk-1-deficient mice. Nature 376: 62-66, 1995). In addition to studies evaluating the process of vascular development, pathologic conditions such as cancer and ischemic heart disease are under intense evaluation to determine whether the control of angiogenic processes can be used to alter pathology progression. During these studies, numerous common pathways to control angiogenesis have been elucidated and can be generally classified in relation to growth factors and their receptors (e.g., VEGF, FGF), methalloproteinases, and extracellular matrix proteins. The formation of a microvasculature in association with an ePTFE based immunoisolation device is accelerated to provide rapid restoration of normal nutrient flow to transplanted islets.

### Prevascularized Constructs for Tissue Perfusion

The microvascular construct is based on a 3-D angiogenesis system comprised of isolated, intact microvessel elements (e.g., arterioles, capillaries and venules) suspended and cultured in a three-dimensional collagen gel. These vessel elements grow in an *in vivo*-like manner, ultimately forming an elaborate collection of microvessels that fill the collagen gel space. The new microvessels (neovessels) that form retain a patent tube structure, maintain associated mural cells and remodel the surrounding collagen matrix. The vessel fragments within this culture system are responsive to the presence of co-cultured cells and exogenous growth factors. Importantly, embedded microvessel fragments in this system perform the same *in vivo* stages of angiogenesis by first elaborating numerous vessel "sprouts". Angiogenesis in the *in vitro* system of the invention begins by day 4 of establishing the cultures and is dynamic, with new sprouts forming and regressing within a day. By 11 days in culture, fragments have grown to form a collection of elongated, simple neovessels with an average diameter of 24.8 ± 6.8 microns (n=39). These neovessels have a patent lumen and a low density of alpha-actin positive, perivascular cells. Unlike with cultured endothelial cells, the vessel fragments of the microvascular construction form new vessels from existing vessels, the hall mark definition of angiogenesis.

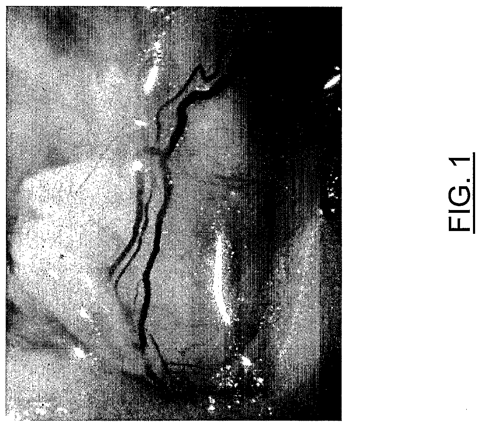

The inventors evaluated the ability of this microvascular construct to interact with an existing vasculature and form a functional microvascular bed by implanting the constructs into immune compromised (SCID) mice. Constructs were placed under the skin and in direct contact with the dorsal musculature. Upon gross examination of implants, the inventors observed superficial, blood-filled vessels associated with microvascular constructs but not with a vascular, collagen gel controls. Histology of explanted, microvascular constructs reveals the presence of blood in the vessels and heterogeneous vessels in the vascular network of the construct. The inventors observed the full range of vessel types commonly seen in a mature, functional vascular bed, including small arteries, arterioles, capillaries, venules and veins. Analysis of inosculation by ink perfusion (Figure 1), revealed that vessels within the construct were continuous with the host vasculature, (and thus capable of carrying blood), as early as one day after implantation. The number of perfusion competent vessels (patent and continuous with the host vasculature) increased rapidly over the next 2 days, very similar to what is observed with full thickness skin grafting in which the graft microcirculation is intact.

### PVID Construction and Evaluation

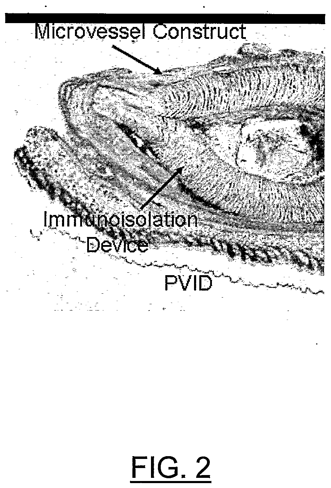

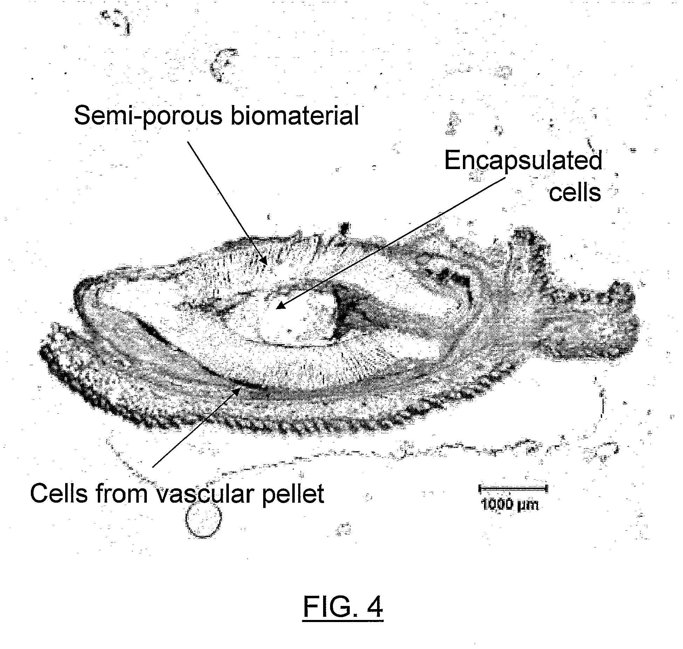

The inventors have established the feasibility of constructing the PVID using an ePTFE immunoisolation device and the isolation fat derived microvessel endothelial cell system described above. Fig. 2 and Fig. 4 illustrate the hybrid system using a synthetic non-degradable ePTFE membrane and a microvessel construct to provide accelerated formation of a mature microcirculation.

### Research Design and Methods

The inventors have evaluated a prevascularized construct composed of a hybrid two-component design using a non-degradable immunoisolation device and a microvascular construct composed of autologous microvessels. Implantation of any device or tissue is at risk for early failure due to the time necessary to recruit and establish a stable vasculature. Methods have been developed to pre-form an adaptive microvascular system capable of rapidly progressing into a mature, efficient perfusion circuit upon implantation. β-islet constructs are prevascularized using this system prior to implantation to accelerate the establishment of a blood supply and preserve β-islet function.

### Microvessel Isolation and Culture

Rat microvessel fragments (MF) are isolated using a modification of previously described methods (Hoying J.B. et al. Angiogenic potential of microvessel fragments established in three-dimensional collagen gels. In Vitro Cell Dev Biol Anim 32: 409-419, 1996; Williams SK and Jarrell BE. Cells derived from omental fat tissue and used for seeding vascular prostheses are not endothelial in origin. J Vasc Surg 15: 457-459, 1992; Williams SK et al. Origin of endothelial cells that line expanded polytetraflourethylene vascular grafts sodded with cells from microvascularized fat. J Vasc Surg 19: 594-604, 1994; Williams SK. et al. Liposuction-derived human fat used for vascular graft sodding contains endothelial cells and not mesothelial cells as the major cell type. Journal of Vascular Surgery 19: 916-923, 1994). Under aseptic conditions, harvested fat tissue is washed in 0.1.% BSA-PBS, finely minced with scissors and digested in 2 mg/ml collagenase + 2mg/ml BSA (essentially fatty acid free) in PBS for 8 min at 37°C with vigorous shaking. Tissue debris and large vessel pieces were removed by filtering the suspension through a sterile 500 µm pore-size nylon screen. Microvessel fragments are captured by filtration of a the remaining suspension on a 30 µm pore size nylon screen and recovered by vigorous flushing of the screen surface with 0.1% BSA-PBS. The type and lot number of collagenase to be used will be pre-screened to optimize fragment yield while maintaining microvessel structure. Microvessel fragments are suspended (12,000 - 15,000) MFs/ml) in ice cold 3 mg/ml rat tail type I collagen (BD BioSciences, Bedford, MA) prepared with DMEM (1x final) and pH-neutralized with 1M NaOH. MF/collagen suspensions will be plated into individual wells (0.25 ml/well) of a 48 well plate and placed in a 37°C incubator for 20 min. to polymerize the collagen.

### Endothelial Pellet Preparation

Epididymal fat was isolated from male rats and digested using collagenase at 37 degrees F. The slurry if digested fat was centrifuged resulting in a floating "cake" of fat cells and a pellet containing endothelial cells. The pellet was resuspended in a mixture of buffer and solubilized collagen. The suspended cells were then placed in a chamber containing a sheet of porous ePTFE on one end. This chamber is produced using a plastic device know as a BEEM capsule. A syringe containing buffer was positioned on the opposite end of the chamber and the inner volume of the chamber was pressurized by advancing the syringe plunger. This resulted in the deposition of the cells from the vascular pellet into the surface of the ePTFE. After 3 days an MTT metabolic assay was performed on the ePTFE sample. The surface of the ePTFE rapidly changed color to a dark purple indicating the presence of viable cells.

### Cell Encapsulation Systems and Implantation

During preliminary studies cell encapsulation devices have been fabricated using ePTFE material. Examples of these devices in illustrated in Figs. 2 and 4. The device's function is evaluated using a mouse model. Mice are anesthetized and prepped for device implantation into the subcutaneous tissue. The device is implanted into the appropriate tissue site. The skin is closed with a metal clip. Device function is based on viability of cells after 1 and 4 weeks of implantation. One potential benefit of this of this approach is the delivery of allografts and xenografts to replace lost cell function as observed. For example, in diabetes, immune deficiency disease and liver disease.

### Vessel Density and Heterogeneity

Intravital fluorescence imaging and histology are used to evaluate vessel organization and heterogeneity surrounding the microvessel/materials implants. Analysis of stained tissue sections (H&E or immunostaining) provides a quantitative measure of vessel elements within the tissue. With this analysis, the number and related presence of capillaries, arterioles and venules for a given area are determined. The origin of the cells in the newly formed microcirculation is evaluated using rat and mouse specific markers to separate host from donor cells.

### Perfusion Competency

Three separate techniques are used to assess the function of the microcirculation formed around the immunoisolation devices: ink perfusion, microangiography and microsphere flow measurements. Ink perfusion and microangiography permit identification of continuous vessel segments, in continuity with the host vasculature, which are patent and competent to carry blood. The perfusion index, using microspheres, quantitatively assesses blood flow in the newly formed microcirculation.

### Evaluation of the Function of a Prevascularized Construct to Support Chronic Islet Function Following Transplantation

Islet isolation and inoculation into devices. Standard procedures for the isolation of viable islets from rats have been developed (Calafiore R. et al. Grafts of microencapsulated pancreatic islet cells for the therapy of diabetes mellitus in non-immunosuppressed animals. Biotechnol Appl Biochem 39: 156-164, 2004; Chaikof EL. Engineering and material considerations in islet cell transplantation. Annu Rev Biomed Eng 1: 103-127, 1999; De Vos P. et al. Efficacy of a prevascularized expanded polytetrafluoroethylene solid support system as a transplantation site for pancreatic islets. Transplantation 63: 824-830, 1997). These islets are evaluated in the PVID developed in aim 1. The PVIDs are prepared and implanted into the subcutaneous position in SCID mice. A silicone tube that provides access to the internal compartment is exteriorized though a skin incision. The end of the tube is heat sealed and covered with a dressing to reduce contamination. Islet inoculation into the device is performed following 1, 3, 7 and 14 days of device implantation. At this time the exteriorized end of the silicone tube is isolated and cut to permit inoculation of islets. Freshly isolated islets are slowly injected into the interior space of the device, the silicone tube heat-sealed and the tube placed under the skin.

### Assessment of Islet Function

The PVIDs containing islets are explanted following 3, 7, 21 and 60 days implantation in SCID mice (Fig. 7). At the time of explanation the PVIDs and surrounding tissue is separated into two segments. One segment is processed for histology to evaluate cell viability inside the PVID and immunocytochemical evaluation of insulin production by islets. The second sample is subjected to Western blot analysis of insulin.

These experiments establish the ability to create a prevascularized immunoisolation device, and subsequently the function of this device is tested in an *in vivo* model. A SCID mouse model is used to assess cell transplantation as the inventors are using a xernograft transplantation model (i.e., rat cells into a mouse). In a human utilization of this technology, the patient's own fat tissue can be used as a source of microvessel fragments for PVID construction and, thus, no immunosuppression is necessary.

Although exemplary embodiments have been shown and described in detail for purposes of clarity, it will be clear to those of ordinary skill in the art from a reading of the disclosure that various changes in form or detail, modifications, or other alterations to the invention as described may be made without departing from the true scope of the invention in the appended claims. For example, while specific dimensions and materials for the constructs, devices and methods have been described, it should be appreciated that changes to the dimensions or the specific materials comprising the device will not detract from the inventive concept. Accordingly, all such changes, modifications, and alterations should be seen as within the scope of the disclosure.

## Claims

1. An implantable device for providing a biologically active agent to a subject in need thereof, the device comprising a microvessel construct in contact with a biocompatible, semi-permeable pouch, the pouch encapsulating a cell or cells capable of producing the biologically active agent, wherein the microvessel construct comprises isolated microvessel fragments at a time prior to implantation.

2. The device of claim 1, wherein the device is an immunoisolation device.

3. The device of claim 1, wherein the vessels of the microvessel construct are syngenic to the subject.

4. The device of claim 1, wherein the cell or cells are allogenic to the subject.

5. The device of claim 1, wherein the cell or cells are syngenic to the subject.

6. The device of claim 1, wherein the pouch comprises a polymer.

7. The device of claim 6, wherein the polymer is expanded polytetrafluoroethylene (ePTFE).

8. The device of claim 6, wherein the polymer is polyethyleneterephthalate (PET).

9. The device of claim 1, wherein the cell or cells is an insulin secreting cell.

10. The device of claim 9, wherein the insulin secreting cell is a beta-islet cell.

## Patentansprüche

1. Implantierbare Vorrichtung zum Bereitstellen eines biologisch aktiven Stoffs an eine dies benötigende Person, wobei die Vorrichtung ein Mikrogefäßgebilde in Kontakt mit einem biokompatiblen, halbpermeablen Beutel beinhaltet, wobei der Beutel eine Zelle oder Zellen einkapselt, die in der Lage sind, den biologisch wirksamen Stoff zu erzeugen, wobei das Mikrogefäßgebilde isolierte Mikrogefäßfragmente zu einem Zeitpunkt vor der Implantation beinhaltet.

2. Vorrichtung nach Anspruch 1, bei welcher die Vorrichtung eine Immunisolierungsvorrichtung ist.

3. Vorrichtung nach Anspruch 1, bei welcher die Gefäße des Mikrogefäßgebildes syngen für die Person sind.

4. Vorrichtung nach Anspruch 1, bei welcher die Zelle oder Zellen allogen für die Person ist/sind.

5. Vorrichtung nach Anspruch 1, bei welcher die Zelle oder Zellen syngen für die Person ist/sind.

6. Vorrichtung nach Anspruch 1, bei welcher der Beutel ein Polymer beinhaltet.

7. Vorrichtung nach Anspruch 6, bei welcher das Polymer expandiertes Polytetrafluorethylen (ePTFE) ist.

8. Vorrichtung nach Anspruch 6, bei welcher das Polymer Polyethylenterephthalat (PET) ist.

9. Vorrichtung nach Anspruch 1, bei welcher die Zelle oder Zellen (eine) Insulin abgebende Zelle(n) ist/sind.

10. Vorrichtung nach Anspruch 9, bei welcher die Insulin abgebende Zelle eine Beta-Inselzelle ist.

## Revendications

1. Dispositif implantable pour fournir un agent biologiquement actif à un sujet qui en a besoin, le dispositif comprenant une construction de micro-vaisseaux en contact avec une poche semi-perméable biocompatible, la poche encapsulant une cellule ou des cellules disposant de la capacité de produire l'agent biologiquement actif, dans lequel la construction de micro-vaisseaux comprend des fragments de micro-vaisseaux isolés à un instant avant implantation.

2. Dispositif selon la revendication 1, dans lequel le dispositif est un dispositif d'immuno-isolation.

3. Dispositif selon la revendication 1, dans lequel les vaisseaux de la construction de micro-vaisseaux sont syngéniques vis-à-vis du sujet.

4. Dispositif selon la revendication 1, dans lequel la cellule ou les cellules est/sont allogénique(s) vis-à-vis du sujet.

5. Dispositif selon la revendication 1, dans lequel la cellule ou les cellules est/sont syngénique(s) vis-à-vis du sujet.

6. Dispositif selon la revendication 1, dans lequel la poche comprend un polymère.

7. Dispositif selon la revendication 6, dans lequel le polymère est du polytétrafluoroéthylène expansé (ePTFE).

8. Dispositif selon la revendication 6, dans lequel le polymère est du téréphtalate de polyéthylène (PET).

9. Dispositif selon la revendication 1, dans lequel la cellule ou les cellules est/sont une cellule ou des cellules de sécrétion d'insuline.

10. Dispositif selon la revendication 9, dans lequel la cellule de sécrétion d'insuline est une cellule bêta d'îlot(s).
